# EUROPÄISCHE PATENTSCHRIFT

(11) **EP 4 172 434 B1**
(45) Veröffentlichungstag und Bekanntmachung des Hinweises auf die Patenterteilung: **24.04.2024**
(21) Anmeldenummer: 21732814.5
(22) Anmeldetag: 28.05.2021
(51) Int. Cl.: E05B 1/00, A61L 2/18

(54) **TÜRGRIFF MIT BETÄTIGUNGSELEMENT ZUR DESINFEKTION EINER GRIFFFLÄCHE DES TÜRGRIFFS SOWIE TÜRKLINKE UND TÜR**
DOOR HANDLE HAVING ACTUATION ELEMENT FOR DISINFECTING A GRIP SURFACE OF THE DOOR HANDLE, AS WELL AS DOOR HANDLE FITTING AND DOOR
POIGNÉE DE PORTE AYANT UN ÉLÉMENT D'ACTIONNEMENT SERVANT À DÉSINFECTER UNE SURFACE DE PRÉHENSION DE LA POIGNÉE DE PORTE, ET FERRURE DE POIGNÉE DE PORTE ET PORTE

(30) Priorität: 29.06.2020 DE 202020103744 U; 03.02.2021 DE 202021100546 U
(43) Veröffentlichungstag der Anmeldung: 03.05.2023
(73) Patentinhaber: Flaig, Hartmut, 78554 Aldingen (DE)
(72) Erfinder: Flaig, Hartmut, 78554 Aldingen (DE)
(74) Vertreter: Patent- und Rechtsanwälte Behrmann Wagner PartG mbB
(86) Internationale Anmeldenummer: PCT/EP2021/064308
(87) Internationale Veröffentlichungsnummer: WO 2022/002498

(56) Entgegenhaltungen:
- EP-A2- 3 118 395
- DE-A1-102012 017 365
- DE-U1-202004 006 845
- GB-A- 2 436 284
- US-A1- 2014 338 153

## Beschreibung

Die vorliegende Erfindung betrifft einen Türgriff nach dem Oberbegriff des Anspruchs 1, eine Türklinke mit Türdorn und erfindungsgemäßem Türgriff gemäß dem Anspruch 11, einem Stoßgriff nach Anspruch 15 und eine Tür mit Türklinke gemäß dem Anspruch 13 und eine Tür mit einem Stoßgriff nach dem Anspruch 17.

Gattungsgemäße Türklinken bestehen aus einem Türgriff, einem Türdorn und einem Montageelement, um den Türdorn an einem Türblatt zu befestigen. In dem Montageelement kann sich eine Drehfeder als Rückstellelement des Türgriffs befinden, um nach einem manuellen Betätigen, Verdrehen oder Herunterdrücken der Türklinke und Öffnen eines Türschlosses den Türgriff in eine üblicherweise horizontale Ausgangslage zurück zu versetzen.

Alternativ oder zusätzlich zu der Türklinke kann der Türgriff als ein Stoßgriff ausgebildet sein und fest, insbesondere nicht verdrehbar an dem Türblatt montiert sein, wobei die Tür üblicherweise im geöffneten Zustand eines Türschlosses mittels des Stoßgriffes bedienbar ist und ein Rückstellmittel in einem Türscharnier aufweist, um die Tür nach einer Betätigung selbständig zu schließen. Dadurch lässt sich die Tür vorzugsweise insbesondere durch eine reine Stoßbewegung auf den Stoßgriff öffnen. Häufig ist dieser Stoßgriff als ein Art Bügelgriff an dem Türblatt montiert.

Insbesondere bei Innen und/oder Außenraumtüren in öffentlichen Einrichtungen, wie Schulen und Krankenhäusern, und/oder Unternehmen in denen Türen von einer hohen Anzahl unterschiedlicher Bedienpersonen händisch betätigt werden, besteht die Gefahr der Übertragung von potentiellen gesundheitsgefährlichen Erregern oder Keimen über den Kontakt mit dem Türgriff. Besonders in diesen Einrichtungen werden eine regelmäßige Desinfektion des Türgriffs und die gleichzeitige Desinfektion der Handfläche der Bedienpersonen gefordert.

Aus der US 2014/338153 A1 ist eine Vorrichtung zur Desinfektion eines manuell betätigbaren Türgriffes bekannt, der einen Behälter für ein flüssiges Desinfektionsmittel aufweist. Der Türgriff ist mit einem ein Desinfektionsmittel speichernden Element in Form einer Hülle oder Hülse gebildet, die auf die Außenfläche des Griffes aufgeschoben ist. Das Desinfektionsmittel speichernde Element besteht aus einem schwammartigen Material, das die Eigenschaft besitzt, ein Desinfektionsmittel zwischenzuspeichern und dieses bei Anwendung einer äußeren Druckkraft abzugeben. Sobald eine Bedienperson Druck auf das das Desinfektionsmittel speichernde Element ausübt, wird Desinfektionsmittel durch Poren des Elementes abgegeben und dessen Außenfläche benetzt, wodurch es zu einer Desinfektion sowohl der Grifffläche als auch der Hand der Bedienperson, die den Griff berührt, kommt.

Die DE 10 2012 017 365 A1 offenbart ebenfalls eine Vorrichtung zur Desinfizierung eines Türgriffs bei Betätigung der Türklinke. Der Türgriff der Türklinke weist innen einen Hohlraum auf, der mit Desinfektionsmittel gefüllt ist. Der Hohlraum ist durch Bohrungen mit einer Umhüllung aus Schaumstoff oder schwammartigen Material verbunden. Wird ein Druckkraft durch die Finger einer Hand auf den Schaumstoff ausgeübt, so wird dieser zusammengedrückt und das Desinfektionsmittel wird aus dem Schaumstoff nach außen gedrückt und kommt so mit der gesamten Hand in Berührung.

Die GB 2 436 284 A zeigt einen Türgriff der im Gegensatz zu einer Türklinke fest als Stoßgriff auf einem Türblatt montiert ist. Der Türgriff weist ein schaumstoffartiges Element auf, das einen Hohlraum innerhalb des Türgriffs ausfüllt und teilweise die Oberfläche des Türgriffs ausbildet. Ein Desinfektionsmittelbehälter ist oberhalb des Hohlraums in dem Stoßgriff integriert und der Stoßgriff ist derart an dem Türblatt montiert, dass das Desinfektionsmittel selbständig aus dem Behälter in das schaumstoffartige Element fließt. Durch Betätigung und ein Greifen des Türgriffs wird der Schaumstoff komprimiert und das Desinfektionsmittel in die Hand einer Bedienperson abgegeben.

Schwammartige Materialien oder Schaumstoffe weisen üblicherweise eine unregelmäßige Oberfläche mit einer Vielzahl an geöffneten Poren auf. Solche Oberflächen oder Materialien sind jedoch nur bedingt für einen hygienischen Einsatz geeignet, da Verunreinigungen und Keime sehr leicht in den Poren haften bleiben. Insbesondere die Reinigung der Oberfläche bis in die Poren ist aufwändig, sodass unter Umständen der gesamte Schaumstoff regelmäßig demontiert und ausgetauscht werden muss. Des Weiteren ist die Menge der Abgabe des Desinfektionsmittels schwer einstellbar und eine ungleichmäßig verteilte Druckkraft auf die Grifffläche begünstigt einen ungleichmäßigen sowie einen stellenweise erhöhten Austritt an Desinfektionsmittel. Dadurch wird eine Tropfenbildung begünstigt, wodurch sich in unerwünschterweise Flüssigkeit auf dem Boden unterhalb des Türgriffs ansammeln kann. Weiterhin muss in kurzen sowie unregelmäßigen Abständen der Behälter des Desinfektionsmittels aufgefüllt werden.

Die EP 3 118 395 B1 beschreibt ein Abdeckelement oder eine Art Schlauch mit Öffnungsspalten, das auf einem bereitgestellten Türgriff angeordnet werden kann, wobei das Abdeckelement ebenfalls mit einem Behälter für Desinfektionsmittel verbunden ist. Sobald eine Bedienperson eine Druckkraft auf das Abdeckelement ausübt, insbesondere während des Betätigen einer Türklinke mittels des Türgriffs, werden die als Ventil wirkenden Öffnungsspalten geöffnet und das Desinfektionsmittel fließt unter dem Einfluss der Schwerkraft aus einem Behälter in einen Griffbereich des Türgriffs.

Weiterhin offenbart die DE 20 2004 006 845 U1 einen Türgriff mit einem Hohlraum zur Leitung eines Desinfektionsmittels, wobei der Türgriff aus einem Kunststoff besteht und Klappen als Betätigungselemente aufweist, die mittels einer Druckkraft einer Bedienperson zusammengedrückt werden können, sodass das Desinfektionsmittel im Inneren des Türgriffs unter Einfluss der Schwerkraft in die Grifffläche fließt.

Die eben genannten als Ventil wirkenden Betätigungselemente ermöglichen zwar das fließen von Desinfektionsmittel in den Bereich der Grifffläche eines Türgriffs, jedoch wird der Zufluss von weiterem Desinfektionsmittel während dem Betätigen des Türgriffs nicht gestoppt. So ist die Abgabe von Desinfektionsmittel abhängig von der Zeit der Betätigung. Dadurch ergibt sich ein unregelmäßiger und unter Umständen übermäßiger Verbrauch an Desinfektionsmittel mit unerwünschter Tropfenbildung und Verbrauchserhöhung ohne Mehrwert bezüglich der Desinfektionswirkung. Auch hier ist eine porenhafte Grifffläche oder eine Grifffläche mit verschiedenen Vertiefungen anfällig für Verunreinigungen und weist eine schwer reinigbare Oberfläche auf. Insbesondere falls zusätzlich ein schwammartiges Material innerhalb des Türgriffs angeordnet ist, können Verunreinigungen nur schwer gereinigt oder das Material regelmäßig ersetzt werden.

Der vorliegenden Erfindung liegt die Aufgabe zugrunde, einen Türgriff vorzuschlagen, der bei Vermeidung der aus dem Stand der Technik bekannten Probleme bei einer Betätigung der Türklinke die Grifffläche des Türgriffs und gegebenenfalls auch die Hand eines Benutzers oder einer Bedienerperson desinfiziert.

Diese Aufgabe wird mit einem Türgriff mit den Merkmalen des unabhängigen Anspruchs 1, mit einer Türklinke gemäß dem Anspruch 11 und einer Tür gemäß dem Anspruch 13 gelöst.

Vorteilhafte Ausführungen sind Gegenstand der Unteransprüche.

Erfindungsgemäß wird ein Türgriff, insbesondere für Innen und/oder Außenraumtüren in öffentlichen Einrichtungen und/oder Unternehmen, mit Betätigungselement zur Desinfektion einer Grifffläche des Türgriffs vorgeschlagen, wobei das Betätigungselement zumindest teilweise in dem Türgriff angeordnet ist und in einer nicht betätigten ersten Stellung einen Hohlraum im Inneren des Türgriffs begrenzt, wobei der Türgriff von einem Kanal durchdrungen ist, der einends in den Hohlraum mündet und andernends Verbindungsmittel zur Verbindung mit einem Behälter aufweist, um über den Kanal und die Verbindungsmittel eine Flüssigkeit, insbesondere ein Desinfektionsmittel, von dem Behälter in den Hohlraum zu leiten, und wobei das Betätigungselement entgegen zumindest ein Rückstellmittel manuell verschiebbar ist. Dabei ist der Hohlraum mit zumindest einem Dichtelement zwischen einer ersten Dichtfläche des Betätigungselements und einer zweiten Dichtfläche des Türgriffs abgedichtet, wobei eine Aussparung in zumindest einer der Dichtflächen ausgebildet ist, um in einer das Volumen des Hohlraums gegenüber der ersten Stellung verkleinernden zweiten Stellung des Betätigungselements das Dichtelement von zumindest einer Dichtfläche zu lösen und um die Flüssigkeit aus dem Hohlraum in Richtung der Grifffläche zu verdrängen, wobei ein Versperrabschnitt der ersten Dichtfläche so an einem Übergang zwischen dem Hohlraum und dem Kanal angeordnet und ausgerichtet ist, dass der Versperrabschnitt des Betätigungselements den Kanal bei der Überführung des Betätigungselements von der ersten in die zweite Stellung zumindest teilweise schließt oder versperrt und wobei in einer das Volumen des Hohlraums gegenüber der zweiten Stellung weiter verkleinernden dritten Stellung des Betätigungselements das Dichtelement an beiden Dichtflächen anliegt, um den Kanal und/oder den Hohlraum vollständig zu schließen.

Dabei hat die Erfindung erstaunlicherweise erkannt, dass durch die erfindungsgemäße Ausführung des Türgriffs nach einem Bedienen des Betätigungselements eine definierte Menge an Desinfektionsmittel in die Grifffläche fließt. Dabei wirkt das Betätigungselement gleichzeitig als Ventil zum Öffnen und Schließen des Kanals und dem Zulauf an Desinfektionsmittel und als eine Art Verdrängerpumpe, um die Flüssigkeit aus dem Hohlraum in die Grifffläche zu befördern. Das Betätigungselement benötigt also nicht die Schwerkraft, um eine Flüssigkeit aus dem Hohlraum in die Grifffläche zu befördern. Vorzugsweise wird die Schwerkraft ausschließlich für das Nachfüllen des Hohlraums nach einem Betätigungsvorgang benötigt, insbesondere wenn sich das Betätigungselement in der ersten Stellung befindet oder nach einer Betätigung oder Verstellung wieder in die erste Stellung zurückkehrt. Mit dieser Ausführung wird also ein definiertes Flüssigkeitsvolumen aus dem Hohlraum in die oder auf die Grifffläche gepumpt und zum Beispiel ein Tropfen von übermäßiger Flüssigkeit auf den Boden kann verhindert werden. Der Türgriff mit dem Betätigungselement weist also eine Art Auslaufsicherung bei einer Dauerbetätigung der Türklinke oder des Betätigungselements auf. Durch die definierte Abgabe an Flüssigkeit kann weiter die Häufigkeit eines Wiederauffüllens des Behälters reduziert werden oder ein kleinerer Behälter kann eingesetzt werden. Insgesamt wird also ein effizienter Einsatz der Flüssigkeit ermöglicht.

Des Weiteren ist der bevorzugt zweiteilige Türgriff mit Betätigungselement einfach montierbar und fertigbar. Es müssen zum Beispiel keine weiteren Ventilelemente mit Drahtzügen oder schwammartigen Schaumstoffe oder andere komplexe Stellmechanismen verbaut werden, die nicht nur die Fertigung komplexer gestalten, sondere insbesondere auch schlecht reinigbar sind und ein Anhaften von Keimen begünstigen können. Außerdem weist die Grifffläche keine Vielzahl an Poren oder Vertiefungen auf und schafft so eine für ein Keimwachstum ungünstige Umgebung die besonders leicht zu reinigen ist.

Gemäß einer bevorzugten Ausführungsform ist das Betätigungselement derart zumindest teilweise in dem Türgriff angeordnet, dass in einem an einem Türblatt montierten Zustand des Türgriffs eine das Volumen des Hohlraums verkleinernde Stellbewegung des Betätigungselements in Richtung der Schwerkraft ausführbar ist. In diesem Fall ist der Türgriff bevorzugt horizontal und senkrecht zu der Richtung der Schwerkraft ausgerichtet. Dadurch wird erreicht, dass bei dem Bedienen und Drücken des Türgriffs in eine zweite Stellung gleichzeitig das Betätigungselement verstellt wird und die Flüssigkeit gleichmäßig aus dem Hohlraum durch das Betätigungselement verdrängt wird und sich gleichmäßig auf der Grifffläche verteilt. Die Flüssigkeit wird vorzugsweise gleichmäßig entlang des gesamten Umfangs des Betätigungselements zwischen den Dichtflächen des Betätigungselements und des Türgriffs hindurch verdrängt.

Alternativ kann der Türgriff auch in einer anderen Orientierung an dem Türblatt angeordnet sein. Dann wäre es ebenfalls möglich das Betätigungselement zu verstellen, indem eine Hand der Bedienperson den Türgriff beidseitig im Bereich des Betätigungselements und der dem Betätigungselement gegenüberliegenden Seite des Türgriffs greift und die Hand zusammendrückt.

In einer weiter bevorzugten Ausführungsform ist der Behälter mit einer Auslassseite oder einem Auslassabschnitt auf dem Türgriff flüssigkeitsdicht in Richtung der Schwerkraft verbunden, bevorzugt verschraubt oder aus Sicherheitsgründen mit dem Türgriff verklebt oder verschweißt, bevorzugt mittels Laserschweißen. Durch diese bevorzugte Anordnung des Behälters in Richtung der Schwerkraft wird gewährleistet, dass Flüssigkeit mittels der Schwerkraft in den Kanal und den Hohlraum fließen kann. Der Behälter ist dabei bevorzugt an dem Türgriff selbst und einem dem Türdorn zugwandten Bereich des Türgriffs angeordnet. Wie bereits beschrieben ist der Türgriff und das Betätigungselement in ihrer Funktion so ausgelegt, dass während des Betätigen des Betätigungselements gerade nur ein Flüssigkeitsvolumen das etwa dem Volumen des Hohlraums entspricht in die Grifffläche gelangt, wobei ein weiteres Nachlaufen an Flüssigkeit aus dem Behälter verhindert wird. Dadurch ergibt sich ein besonders sparsamer Gebrauch an Desinfektionsmittel und es können relativ kleine Behälter, bevorzugt kleiner als 100ml bis 150ml verwendet werden. Solch ein kleiner Behälter ist für bis zu 100 bis 150 Betätigungszyklen geeignet und muss selten aufgefüllt werden.

Weiter bevorzugt ist das Dichtelement ein elastischer O-Ring, der in einer Nut in einer der Dichtflächen des Betätigungselements oder des Türgriffs eingepasst ist, wobei die Aussparung und die Nut in jeweils einer der Dichtflächen des Betätigungselements oder des Türgriffs ausgebildet sind. Insbesondere in der zweiten Stellung des Betätigungselements liegt die Aussparung der Nut gegenüber, sodass sich der Abstand der Dichtflächen zueinander im Vergleich zu der ersten Stellung des Betätigungselements vergrößert und der O-Ring sich von zumindest einer der Dichtflächen löst und ein Verdrängen von Desinfektionsmittelflüssigkeit aus dem Hohlraum in die Grifffläche ermöglicht.

Besonders bevorzugt handelt es sich bei dem O-Ring um einen lösungsmittelbeständigen O-Ring, zum Beispiel aus Nitril- oder Fluor-Karbonkautschuk.

In der ersten Stellung des Betätigungselements ist bevorzugt vorgesehen, dass eine Haftkraft zwischen dem Dichtelement und den Dichtflächen größer ist als die Kraft des Rückstellmittels, um eine klemmende Befestigung des Betätigungselements im Türgriff zu erreichen. Es entsteht also zumindest gerade ein Gleichgewicht zwischen der Kraft des Rückstellmittels und der Haftkraft des Dichtelements an den Dichtflächen. In der Montage oder der Demontage ist lediglich die Haftkraft des Dichtelements zu überwinden, um das Betätigungselement in den Hohlraum des Türgriffs einzupassen oder aus dem Türgriff zu lösen. Zur Montage kann das Betätigungselement bevorzugt per Hand in den Türgriff geschoben und in die erste Stellung gedrückt werden. Zur Demontage kann über den Kanal, zum Beispiel mit Druckluft, ein Überdruck erzeugt werden, um das Dichtelement von den Dichtflächen zu lösen. Anstelle der Druckluft kann auch mit einem Werkzeug das Betätigungselement im Bereich der Grifffläche des Betätigungselements geklemmt und aus dem Türgriff gezogen werden. Die Haftkraft wird bevorzugt so oder so groß gewählt, dass die oben beschriebene Selbsthaftung über eine Vielzahl von Betätigungszyklen erhalten bleibt selbst wenn das Dichtelement einen geringfügigen Verschleiß erfährt.

Mit anderen Worten ist bevorzugt vorgesehen, dass das Betätigungselement durch das Dichtelement, insbesondere ein O-Ring, alleine innerhalb des Türgriffs fixiert werden kann und vorzugsweise keine weiteren Befestigungsmittel zur Befestigung des Betätigungselements notwendig sind.

Alternativ kann das Betätigungselement auch mit einem Befestigungsstift oder einem anderen geometrischen Anschlag in der ersten Stellung entgegen die Kraft des Rückstellelements befestigt sein, jedoch wird dann die Teilezahl des Türgriffs erhöht und die Montage oder die Demontage sowie die Fertigung unnötig komplexer gestaltet. Des Weiteren können sich Rückstände in den Bereichen des Befestigungsstifts oder des geometrischen Anschlags sammeln, sodass eine Reinigung oder die hygienischen Eigenschaften des Türgriffs beeinträchtigt sind.

Als Rückstellelemente oder Rückstellmittel sind vorzugsweise mehrere Federn, besonders bevorzugt zwei Federn, ganz besonders bevorzugt drei Federn, insbesondere Wendelfedern, vorgesehen, die jeweils in einer Vertiefung, insbesondere in einem Sackloch, in dem Betätigungselement geführt sind. Dabei handelt es sich bei der Feder bevorzugt um eine Druckfeder.

Der Türgriff ist vorzugsweise als zylindrischer Rundkörper mit einem bevorzugten Durchmesser zwischen 18mm und 22mm ausgeführt, wobei der Hohlraum bevorzugt als Langloch in dem Türgriff mit einer Länge zwischen 65mm bis 75mm und einer Breite zwischen 10mm bis 14mm ausgebildet ist. Im montierten Zustand des Betätigungselements ist ein Spalt zwischen den Dichtflächen des Betätigungselements und des Türgriffs außerhalb der Aussparung bevorzugt 0.1mm bis 0.5mm breit. Dabei ist das Betätigungselement bevorzugt entsprechend der Form des Langlochs ausgebildet, wobei die Dimensionen des Betätigungselements so gewählt sind, dass der Spalt zwischen den Dichtflächen eingestellt ist.

Das Volumen des Hohlraums in der ersten Stellung des Betätigungselements entspricht bevorzugt 2.2cm³ bis 3cm³. Ein solcher Hohlraum stellt gerade ausreichend Desinfektionsmittel bereit, um die Grifffläche zu benetzen und bevorzugt auch die Handfläche einer Bedienperson. Dabei ist das Volumen klein genug, um eine Tropfenbildung in der Grifffläche zu verhindern.

Weiter bevorzugt sind der Türgriff und das Betätigungselement aus einem Metall, bevorzugt Aluminium oder Edelstahl oder alternativ auch eine Kupferlegierung, gefertigt. Bevorzugt werden antibakteriell oder antimikrobiell wirkende Metalle eingesetzt, um eine für das Wachstum von Keimen und Krankheitserregern ungünstige Umgebungsbedingung zu schaffen. Außerdem sind Türgriffe aus Metall vorteilhafterweise besonders beständig gegenüber einer Vielzahl an antimikrobiellen Reinigungsmitteln. Als Alternative kann der Türgriff und/oder das Betätigungselement auch aus einem Kunststoff mit einer bevorzugt antibakteriellen oder antimikrobiellen Oberfläche oder Oberflächenbeschichtung, bevorzugt in einem Spritzgussverfahren, hergestellt sein. Dadurch sind Türgriffe zwar in großer Stückzahl herstellbar, jedoch ist die chemikalische Beständigkeit unter Umständen reduziert und die Oberfläche muss zusätzlich bearbeitet werden, um eine ungünstige Umgebung für ein Keimwachstum zu schaffen. In einer bevorzugten Ausführungsform weist die Grifffläche eine abrasiv behandelte raue Oberfläche auf, bevorzugt mittels Sand- oder Kugelstrahlen, mit einem Rauheitswert zwischen 20µm und 40µm gemessen nach ISO 8503, um den Kontaktwinkel zwischen der Grifffläche und einem Desinfektionsmittel zu vergrößern. Gemäß der ISO 8503 wird unter dem Rauheitswert die mittlere maximale Höhe zwischen Oberflächenberg und -tal (mean maximum peak-to-valley height) verstanden. Dadurch wird erreicht, dass das Desinfektionsmittel die Grifffläche besser benetzt und eine Tropfenbildung vermieden wird. Eine Flüssigkeit bleibt deshalb länger auf der Grifffläche und wirkt so vorteilhaft für die Desinfektion der Grifffläche.

Die Erfindung umfasst vorzugsweise auch eine Türklinke mit Türdorn und erfindungsgemäßem Türgriff mit Betätigungselement, wobei der Türgriff bevorzugt rechtwinklig an dem Türdorn montiert ist und gegen ein Verdrehen um eine Längsmittelachse des Türgriffs verschraubt ist.

Bevorzugt entspricht die Druckkraft zur Betätigung der Türklinke, insbesondere die Kraft zur Verformung einer Drehfeder im Montageelement des Türdorns, wenigstens einer Kraft, um das Betätigungselement entgegen die Kraft der Rückstellelemente in die dritte Stellung zu verschieben. Dadurch wird gewährleistet, dass bei einem Betätigen der Türklinke das Betätigungselement den Hohlraum und/oder den Kanal in der dritten Stellung schließt und so ein weiterer nicht gewünschter Zulauf an Flüssigkeit aus dem Behälter unterbrochen ist.

Besonders bevorzugt ist die Türklinke also zweiteilig mit Türgriff und Türdorn ausgebildet, wobei der Türgriff, vorzugsweise mit dem Behälter, lösbar an dem Türdorn montiert werden kann. Vorteilhafterweise kann der Türgriff, bevorzugt mit Behälter, so einfach und schnell montiert und/oder demontiert, nachgerüstet, ausgewechselt und/oder gereinigt oder desinfiziert werden, wobei der Türdorn dabei bevorzugt an der Tür montiert verbleiben kann.

Weiterhin umfasst die Erfindung auch eine Tür mit einer bereits beschriebenen Türklinke, wobei der Türgriff ein- oder beidseitig einer Tür montiert ist. Insbesondere eine lösbare Verbindung des Türgriffs mit dem Türdorn ermöglicht eine Montage an beiden Seiten einer Tür, im Gegensatz zu einer einteiligen Ausführung eines Türgriffs mit Türdorn.

Außerdem ist es denkbar, dass ein Behälter an dem Türblatt der Tür angebracht ist und mittels eines Verbindungselementes, insbesondere eines Schlauches, mit dem Kanal des Türgriffs verbunden ist.

Zusätzlich oder alternativ zu der Türklinke, kann der Türgriff mit Betätigungselement und ein Stoßgriff, insbesondere ein Bügelgriff, zur Betätigung einer Tür vorgesehen sein.

Dazu weist der Stoßgriff Aufnahmemittel auf, die zusammen mit dem Türgriff eine bevorzugt zylindrische oder rohrförmige Griffstange mit einer gemeinsamen Erstreckungsachse ausbilden, wobei die Aufnahmemittel als ein erster rohrförmiger Aufnahmeabschnitt den Behälter zur Aufnahme der Flüssigkeit ausbilden, der mit dem Verbindungsmittel des Türgriffs verbunden ist. Vorteilhafterweise kann durch diese Integration des Behälters in die Griffstange die Teileanzahl des Stoßgriffes verringert, sowie die Montage oder eine Nachrüstung von bestehenden Stoßgriffen vereinfacht werden. Es ist also vorzugsweise kein separater Behälter mit Schlauchverbindungen an dem Türblatt notwendig. Des Weiteren kann vorteilhafterweise eine Bedienperson den Stoßgriff auch im Bereich der Aufnahmemittel bedienen. Der so ausgebildete Behälter bildet also auch einen durch eine Bedienperson greifbaren Abschnitt des Stoßgriffs aus.

Vorzugsweise ist der Türgriff, insbesondere als ein zylindrischer Rundkörper, einends mit dem ersten rohrförmigen Aufnahmeabschnitt und andernends mit einem zweiten rohrförmigen Aufnahmeabschnitt einsteckbar verbunden, wobei das bereits beschriebene Verbindungsmittel des Türgriffs umfangsseitig Dichtmittel aufweist, um den ersten rohrförmigen Aufnahmeabschnitt als Behälter abzudichten. Das Verbindungsmittel mit dem Kanal zur Leitung der Flüssigkeit aus dem Behälter in den Hohlraum des Türgriffs ist vorzugsweise entlang der Erstreckungsachse der Griffstange als ein querschnittsreduzierter und zylindrischer Verbindungsabschnitt des Türgriffs ausgebildet, wobei die Dichtmittel entlang des Umfangs des zylindrischen Verbindungsabschnitts angeordnet sind.

Vorzugsweise weist der zylindrische Türgriff, die zylindrischen Aufnahmemittel und dadurch auch die Griffstange einen identischen Durchmesser auf. Vorteilhafterweise ergeben sich daraus keine Querschnittssprünge zwischen dem Türgriff und den Aufnahmemitteln, sodass die Griffstange besonders einfach reinigbar ist und sich keine Verunreinigung in Querschnittssprüngen ansammeln können. Die Verbindungsmittel des Türgriffs sind dabei im montierten Zustand innerhalb der Aufnahmemittel angeordnet.

Als Dichtmittel sind vorzugsweise O-Ringe in einer Führungsnut im Außenumfang der Verbindungsmittel, insbesondere des zylindrischen Verbindungsabschnitts, des Türgriffs angeordnet, wobei im montierten Zustand die O-Ringe derart gegen eine Innenseite des ersten rohrförmigen Aufnahmeabschnittes gepresst werden, dass der Behälter in Richtung des Türgriffs abgedichtet ist und vorzugsweise gleichzeitig der Türgriff kraftschlüssig mit dem ersten Aufnahmeabschnitt verbunden ist. Dadurch lässt sich der Türgriff einfach, insbesondere ohne eine Schraubverbindung montieren und gleichzeitig der Behälter einends abdichten. Andernends ist der Behälter zur Wiederbefüllung mit einem vorzugsweise steckbaren Deckel verschlossen.

Alternativ kann der Deckel mittels einer Schraubverbindung, vorzugsweise mit einem Dichtring, den Behälter schließen und abdichten.

Vorzugsweise weist der Deckel einen Schließmechanismus oder ist vorzugsweise mit einem Spezialwerkzeug oder Spezialschlüssel betätigbar, um einen Diebstahlschutz zu gewährleisten oder ein ungewolltes Befüllen des Behälters zu verhindern. Der Schließmechanismus kann als ein mit einem Schlüssel betätigbares Schloss innerhalb des Deckels ausgebildet sein, wobei ein mit dem Schlüssel drehbares Sicherungselement im geschlossenen Zustand mit dem Behälter formschlüssig, insbesondere in einer Auskerbung der Behälterinnenseite, oder kraftschlüssig mit dem Behälter verbunden ist. Für die Verwendung eines Spezialwerkzeuges weist der Deckel bevorzugt in dessen äußeren Stirnseite Einkerbungen, insbesondere zwei Bohrungen, auf, wobei das Spezialwerkzeug dazu ausgebildet ist in diese Einkerbungen formschlüssig einzugreifen und den vorzugsweise verschraubten Deckel zu betätigen, insbesondere zu rotieren.

Bevorzugt ist dieser abschließbar oder diebstahlsichere Deckel für einen Behälter des Stoßgriffs ausgebildet, sowie für einen Behälter der auf einer Türklinke oder dem Türgriff selbst montiert ist.

Bevorzugt ist der Türgriff mit einem weiteren zylindrischen Verbindungsabschnitt zusätzlich in den zweiten Aufnahmeabschnitt einsteckbar, um auch eine formschlüssige Verbindung des Türgriffs mit dem Türblatt in einem montierten Zustand zu gewährleisten. Dabei sind der erste und der zweite Aufnahmeabschnitt derart ausgebildet, dass der Türgriff mittig der Griffstange angeordnet ist.

Die Erfindung umfasst weiterhin eine Tür mit zumindest einem bereits beschriebenen Stoßgriff, wobei die zylindrische Griffstange des Stoßgriffs mit zumindest einem Montageelement an den Aufnahmemitteln ein- oder beidseitig der Tür montiert ist.

Vorzugsweise ist das Aufnahmemittel als erster und zweiter Aufnahmeabschnitt ausgebildet, wobei jeweils ein Montageelement an dem ersten und dem zweiten Aufnahmeabschnitt angeordnet ist. Durch diese Anordnung bildet der Stoßgriff als Griffstange mit Montageelementen eine Art Bügelgriff aus, wobei vorzugsweise in der Mitte des Bügelgriffs der Türgriff mit dem Betätigungselement angeordnet ist.

Durch die Aufnahmemittel lässt sich also eine Griffstange ausbilden, mit der der Türgriff an das Türblatt montierbar ist und gleichzeitig lässt sich der Behälter platzsparend in der Griffstange integrieren.

Das Betätigungselement des Türgriffs ist vorzugsweise auf einer den Montageelementen gegenüberliegenden Seite der Griffstange derart angeordnet, dass eine Bedienperson durch eine stoßende Betätigung oder die Ausübung einer Stoßkraft oder Druckkraft auf die Griffstange, insbesondere mit der Handfläche, gleichzeitig die Tür bedient oder öffnet und das Betätigungselement mit der Druckkraft gegen die Rückstellelemente betätigt.

Bevorzugt entspricht die Druckkraft zur Betätigung des Stoßgriffs, insbesondere die Kraft zum Öffnen der Tür entgegen Rückstellmittel in den Türscharnieren, wenigstens einer Kraft, um das Betätigungselement entgegen die Kraft der Rückstellelemente in die dritte Stellung zu verschieben. Dadurch wird gewährleistet, dass bei einem Betätigen des Stoßgriffs das Betätigungselement den Hohlraum und/oder den Kanal in der dritten Stellung schließt und so ein weiterer nicht gewünschter Zulauf an Flüssigkeit aus dem Behälter unterbrochen ist.

Alternativ kann das Betätigungselement auch um 180° bezüglich der für eine Druckbewegung bevorzugten Ausrichtung um die Erstreckungsachse der Griffstange gedreht angeordnet sein, wobei dann die Bedienperson das Betätigungselement vorzugsweise mit den Fingern greift und durch eine Zugbewegung an der Griffstange die Tür öffnet und gleichzeitig das Betätigungselement verstellt.

Weiter alternativ kann das Betätigungselement auch zwischen den genannten für eine Zug- oder Druckbewegung bevorzugten Ausrichtungen um die Erstreckungsachse der Griffstange gedreht angeordnet sein, wobei eine seitliche, insbesondere um 90° bezüglich der Ausrichtung für die Druckbewegung gedrehten, Ausrichtung bevorzugt ist. Bevorzugt ist in dieser seitlichen Ausrichtung das Betätigungselement durch eine seitliche Druckkraft auf das Betätigungselement oder durch ein Umgreifen der Griffstange und eine Zusammendrücken der Hand der Bedienperson betätigbar.

Vorzugsweise ist die Erstreckungsachse des Stoßgriffs und die Längsmittelachse des Türgriffs derart ausgerichtet, dass eine Flüssigkeit in dem ersten rohrförmigen Aufnahmeabschnitt mittels der Schwerkraft in den Türgriff fließt, insbesondere durch den Kanal des Verbindungsmittels, wobei die Erstreckungsachse des Stoßgriffs vorzugsweise parallel zu der Richtung der Schwerkraft ausgerichtet ist.

Nachfolgend werden Details und Ausführungsformen der Erfindung anhand von lediglich schematischen Zeichnungen erläutert.

Es zeigen:
- Fig. 1:: eine perspektivische Ansicht einer Türklinke mit Türdorn und Türgriff mit Betätigungselement und Behälter in einem an einem Türblatt montierten Zustand;
- Fig. 2a:: einen Längsschnitt des Betätigungselements und einen Ausschnitt eines Längsschnitts des Türgriffs in einer x-z Ebene gemäß der Fig. 1;
- Fig. 2b:: einen Längsschnitt des Betätigungselements in einer x-z Ebene gemäß der Fig. 2a und eine Seitenansicht des Betätigungselements;
- Fig. 2c:: einen Ausschnitt eines Längsschnitts des Türgriffs in einer x-z Ebene gemäß der Fig. 2a;
- Fig. 2d:: einen Längsschnitt des Betätigungselements und einen Ausschnitt eines Längsschnitts des Türgriffs in einer x-z Ebene gemäß der Fig. 2a und
- Fig 3a bis Fig. 3c:: jeweils eine Detailansicht des Betätigungselements und des Türgriffs gemäß der Fig. 2a in einer ersten, zweiten und einer dritten Stellung des Betätigungselements,
- Fig. 4a: eine Draufsicht auf einen Stoßgriff mit Türgriff in einer x-y Ebene,
- Fig. 4b: eine Schnittansicht des Stoßgriffs gemäß der Fig. 4a entlang einer Schnittachse B-B in einer x-z Ebene,
- Fig. 4c: eine perspektivische Ansicht auf den Stoßgriff gemäß der Fig. 4a und der Fig. 4b.

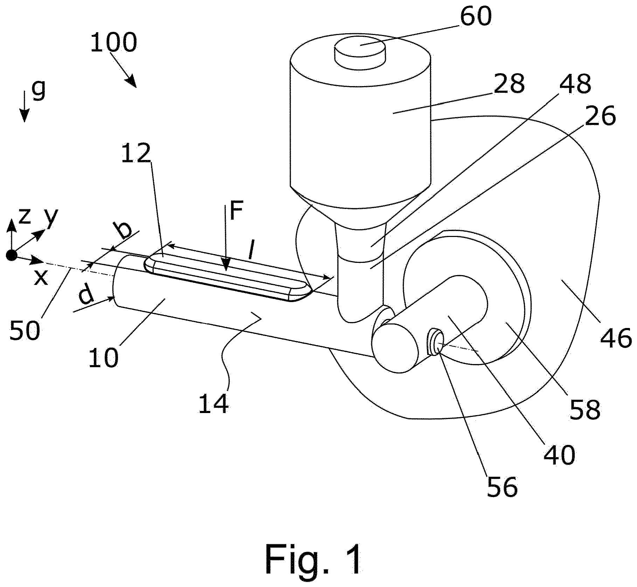

In der Fig. 1 ist eine Türklinke 100 dargestellt, wobei ein Türgriff 10 mit Betätigungselement 12 und ein Behälter 28 an einem Türdorn 40 montiert sind, der wiederum an einem Türblatt 46 montiert ist. Es ist eine nicht betätigte erste Stellung des Betätigungselement 12 dargestellt, wobei bevorzugt ein Teil des Betätigungselements 12 aus dem Türgriff 10, ähnlich einer Taste, herausragt. Die Oberflächen des Betätigungselements 12 und des Türgriffs 10 bilden zusammen eine Grifffläche 14 aus, über die die Türklinke 100 und das Betätigungselement 12 bevorzugt händisch bedient werden können.

Der Türgriff 10 ist in einem an dem Türblatt 46 montierten Zustand in einer nicht betätigten Stellung bevorzugt horizontal angeordnet und das Betätigungselement 12 ist vorzugsweise derart zumindest teilweise in dem Türgriff 10 angeordnet ist, dass eine Stellbewegung des Betätigungselements 12 und eine entsprechende Druckkraft F einer Bedienperson auf die Grifffläche 14 des Betätigungselements 12 in Richtung der Schwerkraft g ausführbar ist. Dadurch lässt sich das Betätigungselement 12 vorzugsweise gleichzeitig mit einem Herunterdrücken der Türklinke 100 bedienen.

Vorzugsweise ist der Behälter 28 mit einer Auslassseite oder einem Auslassabschnitt 48 mit einem Verbindungsmittel 26, bevorzugt mittels einer Schraubverbindung, die mittels Dichtringen abgedichtet ist, mit dem Türgriff 10 verbunden. Bevorzugt ist der Behälter 28 in einer Richtung der Schwerkraft g oberhalb des Türgriffs 10 angeordnet, sodass eine Flüssigkeit in dem Behälter 28 in Richtung des Türgriffs 10 fließt und vorzugsweise kein komplexer Pumpmechanismus erforderlich ist.

Der Behälter 28 ist mit einem Deckel 60 verschlossen, der bevorzugt gegenüber dem Auslassabschnitt 48 angeordnet ist und auf einen Einlassabschnitt aufgeschraubt ist. Vorzugsweise dichtet der Deckel 60 den Behälter 28 ab, um ein Verdampfen des Desinfektionsmittels zu verhindern. Weiter bevorzugt ist der Deckel aus Sicherheitsgründen abschließbar.

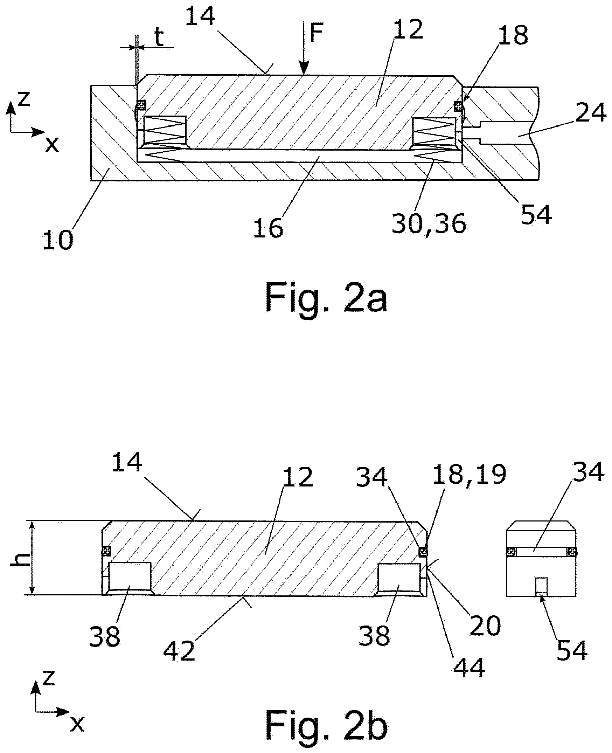

Gemäß der Fig. 2a ist in der nicht betätigten ersten Stellung des Betätigungselements 12 ein Hohlraum 16 im Inneren des Türgriffs 10 durch das Betätigungselement 12 begrenzt. Der Türgriff 10 ist von einem Kanal 24 durchdrungen, der einends in den Hohlraum 16 mündet und andernends mittels eines Verbindungsmittels 26 (siehe Fig. 1) mit einem Behälter 28 verbunden ist, um über den Kanal 24 und die Verbindungsmittel 26 eine Flüssigkeit, insbesondere ein Desinfektionsmittel, von dem Behälter 28 in den Hohlraum 16 zu leiten.

Bevorzugt ist der Kanal 24 entlang einer Längsachse 50 des Türgriffs 10 in den Türgriff 10 eingebracht, zum Beispiel indem der Türgriff 10 von einer dem Türdorn 40 zugewandten Seite aufgebohrt ist und eine entstandene Öffnung klebend oder geschraubt geschlossen ist oder alternativ dort ein Verbindungsmittel, zum Beispiel ein Schlauch, zu einem Behälter 28 angebracht ist. Weiterhin weist das Betätigungselement 12 bevorzugt eine Einkerbung 54 auf, die bevorzugt in der ersten Stellung des Betätigungselements 12 der Öffnung des Kanals 24 gegenüberliegt, sodass in der ersten Stellung des Betätigungselements 12 die Flüssigkeit unbehindert in den Hohlraum 16 fließen kann.

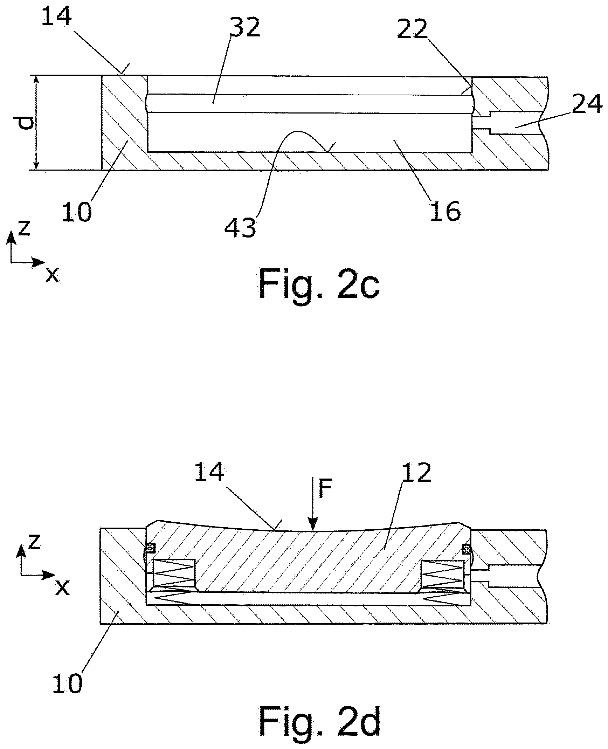

Das Betätigungselement 12 weist im montierten Zustand an den Seitenwänden einen Spalt 52 mit Abstand t zu dem Türgriff 10 auf, wobei sich ein Dichtelement 18 in dem Spalt 52 befindet, um den Hohlraum 16 abzudichten. Insbesondere ist das Dichtelement 18 zwischen einer ersten Dichtfläche 20 des Betätigungselements 12, gemäß der Fig. 2b, und einer zweiten Dichtfläche 22 des Türgriffs 12, gemäß der Fig. 2c, angeordnet.

Gemäß der Fig. 2c ist weiterhin eine Aussparung 32 in der zweiten Dichtfläche 22 des Türgriffs 12 ausgebildet. Alternativ oder zusätzlich kann die Aussparung 32 auch in der ersten Dichtfläche 20 des Betätigungselements 12 ausgebildet sein. Bevorzugt ist diese Aussparung 32 halbkreisförmig und mit einem Kugelkopffräser herstellbar.

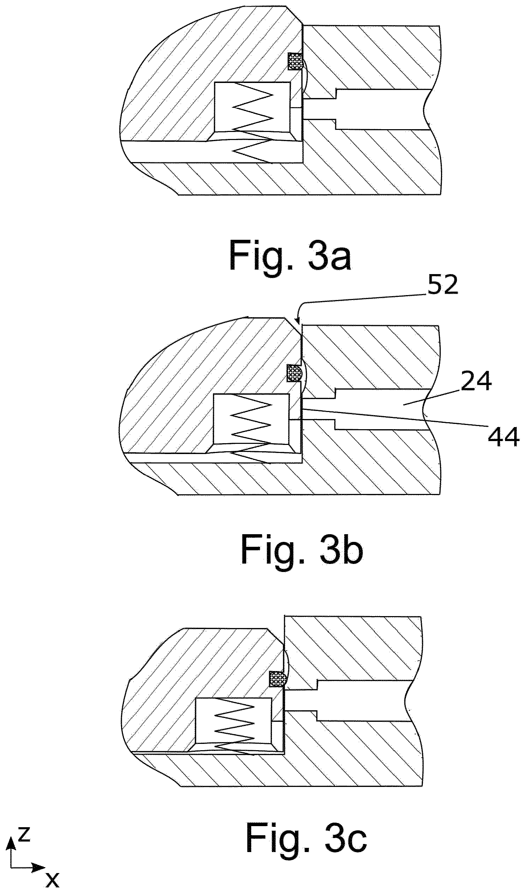

Das Betätigungselement 12 ist entgegen vorzugsweise zwei Rückstellmittel 30 manuell mit einer Druckkraft F bedienbar und ist von einer ersten Stellung in eine zweite und dritte Stellung gemäß den Fig. 3a bis Fig. 3c verschiebbar.

In einer alternativen Ausführungsform kann die Grifffläche 14 des Betätigungselements 12 gemäß der Fig. 2d konkav in Richtung des Hohlraums 16 geformt sein. Dabei ist die konkave Form wenigstens in einer Richtung, bevorzugt in mehreren Richtungen, ausgeführt, um eine Ansammlung an Flüssigkeit in der Grifffläche 14 des Betätigungselements 12 zu begünstigen. Da die konkave Form in Richtung des Hohlraums 16 ausgeführt ist, wird vorteilhafterweise auch ein Abtropfen von der Grifffläche 14 verhindert. Die Menge der Ansammlung an Flüssigkeit entspricht vorzugsweise der Menge an Flüssigkeit die von der Hand einer Bedienperson aufgenommen wird. Die Ansammlung an Flüssigkeit verhindert vorteilhafterweise ein unmittelbares Verdampfen an Desinfektionsmittel bevor dieses von der Hand einer Bedienperson aufgenommen werden kann.

In den Fig. 3a bis Fig. 3b ist der Türgriff 10 mit Betätigungselement 12 in einer Überführung des Betätigungselements 12 von der ersten in eine zweite Stellung dargestellt, wobei in der zweiten Stellung gemäß der Fig. 3b das Volumen des Hohlraums 16 gegenüber der ersten Stellung gemäß der Fig. 3a verkleinert ist. In der zweiten Stellung befindet sich das Dichtelement 18 im Bereich der Aussparung 32. Durch die Aussparung 32 vergrößert sich der Spaltabstand t zwischen der ersten Dichtfläche 20 und der zweiten Dichtfläche 22, wodurch sich das Dichtelement 18 zumindest von einer der Dichtflächen 20, 22 löst. Dadurch ist der Hohlraum 16 teilweise geöffnet und die durch das Betätigungselement 12 verdrängte Flüssigkeit in dem Hohlraum 16 kann durch den Spalt 52 zwischen dem Betätigungselement 12 und dem Türgriff 10 in die Grifffläche 14 fließen.

Durch eine bevorzugte horizontale Montage des Türgriffs 10 an dem Türdorn 40 und einer bevorzugten das Volumen des Hohlraums 16 verkleinernden Stellbewegung des Betätigungselements 12 in Richtung der Schwerkraft g verteilt sich die Flüssigkeit besonders gleichmäßig in der Grifffläche 14 und die Flüssigkeit fließt besonders gleichmäßig zwischen den Dichtflächen 20, 22 entlang des Umfangs des Betätigungselements 12 In der zweiten Stellung des Betätigungselements 12 wird der Kanal 24, bevorzugt gleichzeitig mit dem Öffnen des Hohlraums 16, zumindest teilweise durch einen Versperrabschnitt 44 im Betätigungselement 12 abgedeckt. Dadurch ist der Kanal 24 einends zumindest teilweise geschlossen oder versperrt und der Flusswiderstand wird soweit erhöht, dass ein schwerkraftbedingter Zufluss an Flüssigkeit aus dem Behälter 28 in die Grifffläche 14 zumindest behindert ist.

In der Fig. 3c ist eine dritte Stellung des Betätigungselements 12 dargestellt, in der das Volumen des Hohlraums 16 gegenüber der zweiten Stellung weiter verkleinert ist, wobei sich der Spaltabstand t im Bereich des Dichtelements 18 ebenfalls verkleinert und wobei das Dichtelement 18 wie in der ersten Stellung an beiden Dichtflächen 20, 22 anliegt, um den Kanal 24 und/oder den Hohlraum 16 vollständig zu schließen. In dieser dritten Stellung kann der Türgriff 10 gehalten werden, ohne dass zusätzliche Flüssigkeit schwerkraftsbedingt in die Grifffläche 14 fließt.

In der dritten Stellung liegt das Betätigungselement 12 vorteilhafterweise am Boden 43 oder mit einem geringen Abstand über dem Boden 43 des Hohlraums 16 an, sodass möglichst die gesamte Flüssigkeit durch das Betätigungselement 12 aus dem Hohlraum 16 verdrängt ist und sich dadurch keine verunreinigte Flüssigkeit in dem Hohlraum 16 ansammeln kann.

Das Dichtelement 18 ist vorzugsweise ein elastischer O-Ring 19, der gemäß der Fig. 2a in einer Nut 34 in einer Dichtfläche 20 des Betätigungselements 12 eingepasst ist. Alternativ kann die Nut 34 auch in einer Dichtfläche 22 des Türgriffs 10 angeordnet sein, wobei dann die Aussparung 32 vorzugsweise in der Dichtfläche 20 des Betätigungselements 12 angeordnet ist. Gemäß der Fig. 3a und der Fig. 3c liegt der O-Ring 19 in der ersten und der dritten Stellung des Betätigungselements 12 vorzugsweise teilweise in einem Bereich der Aussparung 32 an der Dichtfläche 22 des Türgriffs an.

Die Rückstellmittel 30 sind bevorzugt so ausgelegt, dass in der ersten Stellung des Betätigungselements 12 gemäß der Fig. 2a oder der Fig. 3a eine Haftkraft zwischen Dichtelement 18 und den Dichtflächen 20, 22 größer ist als eine Kraft des Rückstellmittels 30, um eine klemmende Befestigung des Betätigungselements 12 im Türgriff 10 zu erreichen.

Vorzugsweise sind die Rückstellmittel 30 so vorgespannt, dass zumindest ein Teil des Dichtelements 18 in einen Spalt 52 zwischen den Dichtflächen 20, 22 gedrückt wird. Insbesondere ein elastisches Dichtelement 18, bevorzugt ein O-Ring 19, wird in einem Bereich der Dichtflächen 20, 22 außerhalb der Aussparung 32 in die Nut 34 und den Spalt 52 gedrückt. In diesem Zustand ist die Haftkraft zwischen dem Dichtelement 18 und den Dichtflächen 20, 22 besonders hoch und bevorzugt größer als die Kraft der Rückstellelemente 30. Ein Teil des Dichtelements 18 kann sich dabei in der Aussparung 32 ausdehnen, wodurch sich das Dichtelement 18 zusätzlich durch die geometrische Änderung des Spaltabstandes t im Bereich der Aussparung 32 verklemmt. Das Verklemmen des Dichtelements 18 ermöglicht gleichzeitig die Abdichtung des Hohlraums 16 und die Befestigung des Betätigungselements 12 im Türgriff 10.

Bevorzugt ist der Spaltabstand t oder die Größe und Anordnung des Dichtelements 18 so ausgeführt, dass das Dichtelement 18 zwar durch den Spalt 52 gedrückt und montiert werden kann, allerdings die Dichtwirkung bereits erfolgt, sobald das Dichtelement 18 teilweise an einem Bereich der Aussparung 32 anliegt. Zur Demontage des Betätigungselements 12 ist eine Haftkraft und vorzugsweise auch eine geometrisch bedingte Scherkraft an der Aussparung 32 zu überwinden, um das Dichtelement 18 zu lösen.

Dadurch wird in einer besonders einfachen Weise das Betätigungselement 12 in dem Türgriff 10 fixiert, ohne weitere Fixiermittel verwenden zu müssen.

Als Rückstellmittel 30 werden vorzugsweise zwei Federn 36, insbesondere Wendelfedern, verwendet, die jeweils in einer Vertiefung 38, insbesondere in einem Sackloch, in dem Betätigungselement 12 geführt sind. Dadurch wird eine bevorzugt gleichmäßige Stellbewegung des Betätigungselements 12 ermöglicht und ein Verkanten des Betätigungselements 12 an einer der Dichtflächen 20, 22 verhindert. Deshalb sind die Vertiefungen 38 auch bevorzugt in Längsrichtung äußeren Bereichen des Betätigungselements 12 ausgeführt. Bei der Feder 36 handelt es sich insbesondere um eine Druckfeder die in der ersten Stellung des Betätigungselements 12 vorgespannt ist und durch eine Druckkraft F einer Bedienperson zusammengedrückt werden kann und so als Rückstellmittel wirkt.

Die Kraft der Rückstellmittel 30 sind bevorzugt so gewählt, dass der Übergang von der zweiten Stellung zu der dritten Stellung des Betätigungselements 12 mit geringer manueller Druckkraft F durch eine Bedienperson verstellbar ist, sodass nach einer Bedienung des Betätigungselements 12, insbesondere nach einer üblichen Türklinkenbedienung in der die Türklinke 100 entgegen eine Drehfeder betätigt wird, das Betätigungselement 12 in die dritte Stellung verschoben ist und der Hohlraum 16 wieder verschlossen ist.

Der Türgriff 10 ist bevorzugt aus einem zylindrischen Rundkörper gefertigt, wobei der Durchmesser des Türgriffs 10 zwischen 18mm und 22mm liegt und die Grifffläche 14 des Türgriffs 10 zumindest auf einer Länge von bevorzugt 80mm bis 120mm ausgebildet ist. Dabei wird ein Teilbereich des Türgriffs 10 indem ein Behälter 28 angebracht ist nicht als Teil der Grifffläche 14 betrachtet.

Der Hohlraum 16 ist bevorzugt als Langloch in dem zylindrischen Türgriff 10 ausgeführt und bevorzugt mittig in der Grifffläche 14 angeordnet. Dabei liegt die Länge l des Langlochs bevorzugt zwischen 65mm und 75mm und die Breite b des Langlochs bevorzugt zwischen 10mm bis 14mm. Die Tiefe des Langlochs im Türgriff 10 liegt bevorzugt zwischen 14mm und 17mm.

Die Form des Betätigungselements 12 ist vorzugsweise dem als Langloch ausgeführten Hohlraum 16 angepasst, wobei sich die Länge und Breite des Betätigungselements 12 aus den Dimensionen des Hohlraums 16 unter Abzug des Spaltabstands t ergeben. Die eine Grifffläche 14 ausbildende Oberfläche des Betätigungselements 12 weist bevorzugt abgerundete oder abgeschrägte Kanten auf, sodass in der ersten Stellung des Betätigungselements 12 der Übergang von dem Bereich der Grifffläche 14 des Türgriff 10 in den Bereich der Grifffläche 14 des Betätigungselements 12 bevorzugt kontinuierlich oder mit einer geringen Kantenhöhe verläuft. Die Höhe h des Betätigungselements 12 beträgt bevorzugt 15mm bis 17mm. Bei dieser Höhe h wird gewährleistet, dass sich das Betätigungselement 12 nicht während einer Stellbewegung mit den Innenflächen des Hohlraums 16 verkantet.

Der Spalt t zwischen den Dichtflächen 20, 22 und außerhalb der Aussparung 32 ist vorzugsweise 0.1mm bis 0.5mm breit. Durch die geringe Breite kann in Verbindung mit der erwähnten Höhe h des Betätigungselements 12 eine gute Führung des Betätigungselements 12 und ein Verkanten verhindert werden, sodass das Betätigungselement 12 sich möglichst gleichmäßig verstellt und der Flüssigkeitsaustritt entlang des Umfangs des Betätigungselements 12 gleichmäßig ist.

Des Weiteren ist eine Nut 18 zur Aufnahme des Dichtelements 18 bevorzugt in Umfangsrichtung des Betätigungselements 12 ausgeführt und in einem bevorzugten Abstand von der Unterseite 42 des Betätigungselements 12 zwischen 8mm bis 9mm angeordnet.

Eine Breite der Nut 34 ist vorzugsweise größer als der Durchmesser des O-Rings 19, um eine Verformung des O-Rings 19 innerhalb der Nut zu ermöglichen, sobald dieser zwischen den Dichtflächen 20, 22 eingespannt ist. Der Durchmesser des Querschnitts des O-Rings 19 und die Tiefe der Nut 34 ist abhängig von dem Spaltabstand t zwischen den Dichtflächen 20, 22. Die Spaltabstandvergrößerung im Bereich der Aussparung 32 ist wiederum abhängig von dem Durchmesser des Querschnitts des O-Rings 19. Vorzugsweise ist die Tiefe der Nut 34 so gewählt, dass 20% bis 40% des O-Ringdurchmessers aus der Nut 34 heraussteht. Dabei sollte der Spaltabstand t außerhalb der Aussparung 32 wenigstens kleiner als der Überstand des O-Ringdurchmessers sein, um eine ausreichende Dichtwirkung zu gewährleisten. Zur Montage wird das Betätigungselement 12 mit O-Ring 19 in den Hohlraum 16 gedrückt, wobei der O-Ring 19 sich elastisch verformt. Das Betätigungselement 12 wird soweit verschoben, bis der O-Ring 19 sich im Bereich der Aussparung 32 aufweitet und durch die Rückstellelemente 30 in der ersten Stellung verklemmt. Ein bevorzugter O-Ringdurchmesser beträgt 1.5mm bis 2mm.

Im montierten Zustand des Betätigungselements 12 im Hohlraum 16 liegt ein Abstand zwischen der Unterseite 42 des Betätigungselements 12 und des Bodens 43 des Hohlraums 16 je nach Stellung des Betätigungselements 12 zwischen 0mm bis 3mm. Wie bereits erwähnt ist die Aussparung 32 so angeordnet und dimensioniert, dass das Dichtelement 18 in der ersten und dritten Stellung den Hohlraum 16 abdichtet. Deshalb kann die Dimension und Anordnung der Aussparung 32 von der Anordnung des Dichtelements 18 und dem gewünschten Abstand zwischen der Unterseite 42 des Betätigungselements 12 und des Bodens 43 des Hohlraums 16 bestimmt werden, wobei die Aussparung 32 bevorzugt in einem Abstand von 8mm bis 13mm von dem Boden 43 des Hohlraums 16 eingebracht ist.

Der Versperrabschnitt 44 ist zumindest in einem Bereich der Dichtfläche 20 des Betätigungselements 12 ausgeführt, die einer Öffnung des Kanals 24 in den Hohlraum 16 zugewandt ist. Die Größe des Versperrabschnitts 44 wird durch die Einkerbung 54 im Betätigungselement 12 vorgegeben, wobei eine Höhe der Einkerbung 54 im Betätigungselement 12 so gewählt ist, dass der Auslass des Kanals 24 in den Hohlraum 16 in einer ersten Stellung des Betätigungselements 12 geöffnet ist. Bevorzugt liegt die Höhe der Einkerbung 54 im Betätigungselement 12 zwischen 3mm bis 4mm. Dabei ist weiterhin bevorzugt, dass die Einkerbung 54 einen Durchbruch zu der Vertiefung 38 der Rückstellmittel 30 ausbildet.

Im montierten Zustand des Betätigungselements 12 entspricht das Volumen des Hohlraums 16 in der ersten Stellung des Betätigungselements 12 bevorzugt 2.2cm³ bis 3cm³. Vorzugsweise liegt dann das Flüssigkeitsvolumen, das durch das Betätigungselement 12 in die Grifffläche 14 durch eine Stellbewegung von der ersten Stellung in die dritte Stellung gelangt, zwischen 1ml bis 3ml.

Besonders bevorzugt sind der Türgriff 10 und das Betätigungselement 12 aus einem Metall, bevorzugt Aluminium oder Edelstahl, gefertigt.

Die Grifffläche 14 weist bevorzugt eine abrasiv behandelte raue Oberfläche auf, bevorzugt mittels Kugel- oder Sandstrahlen, mit einem Rauheitswert zwischen 20µm und 40µm gemessen nach ISO 8503, um den Kontaktwinkel zwischen der Grifffläche 14 und einem Desinfektionsmittel zu vergrößern.

Wie in der Fig. 1 dargestellt, ist der Türgriff 10 bevorzugt rechtwinklig an den Türdorn 40 montiert und gegen ein Verdrehen um eine Längsmittelachse 50 des Türgriffs 10 verschraubt. Der Türgriff 10 weist einends einen verjüngten Abschnitt 56 auf, der den Türdorn 40 durchdringt. Vorzugsweise ist der Türdorn 40 hohl ausgeführt und eine Schraube zur Befestigung des Türgriffs 10 ist in ein Gewinde im Inneren des Türdorns 40 in Richtung einer Längsachse des Türdorns 40 auf eine bevorzugt ebene Fläche des verjüngten Abschnitts 56 des Türgriffs 10 schraubbar. Im montierten Zustand des Türdorns 40 an einem Türblatt 46 ist diese Schraube für eine Bedienperson nicht ohne eine Demontage des Türdorns 40 selbst zugänglich, sodass ein gewisser Diebstahlsschutz vorhanden ist.

Besonders bevorzugt entspricht die Druckkraft F auf den Türgriff 10 zur Betätigung der Türklinke 100, insbesondere zur Verformung einer Drehfeder, wenigstens einer Kraft, um das Betätigungselement 12 entgegen die Kraft der Rückstellelemente 30 in die dritte Stellung zu verschieben. Dadurch wird eine Art Auslaufsicherung erreicht, da der Hohlraum 16 und/oder der Kanal 24 in der dritten Stellung des Betätigungselements 12 geschlossen sind. Die Drehfeder kann in einem Montageelement 58 der Türklinke 100 angeordnet sein, wobei die Drehfeder durch eine relative Drehbewegung zwischen Türdorn 40 und Montageelement 58 gespannt wird.

Wie ebenfalls aus der Fig. 1 ersichtlich, kann der Türgriff 10 ein- oder beidseitig einer Tür montiert sein, da die Türklinke 100 aus einem demontierbaren Türgriff 10 und Türdorn 40 besteht. Solange ein Türdorn 40 mit entsprechender Montageaufnahme bereitgestellt ist, kann der Türgriff 10, im Gegensatz zu einer Türklinke 100 mit einem einstückigen Türdorn 40 und Türgriff 10, in einer beliebig gedrehten Ausrichtung um die Längsachse 50 des Türgriffs 10 angeordnet sein.

In einer weiteren Ausführungsform ist ein Behälter 28 an dem Türblatt 46 angebracht und mittels eines Schlauches mit dem Kanal 24 des Türgriffs 10 verbunden.

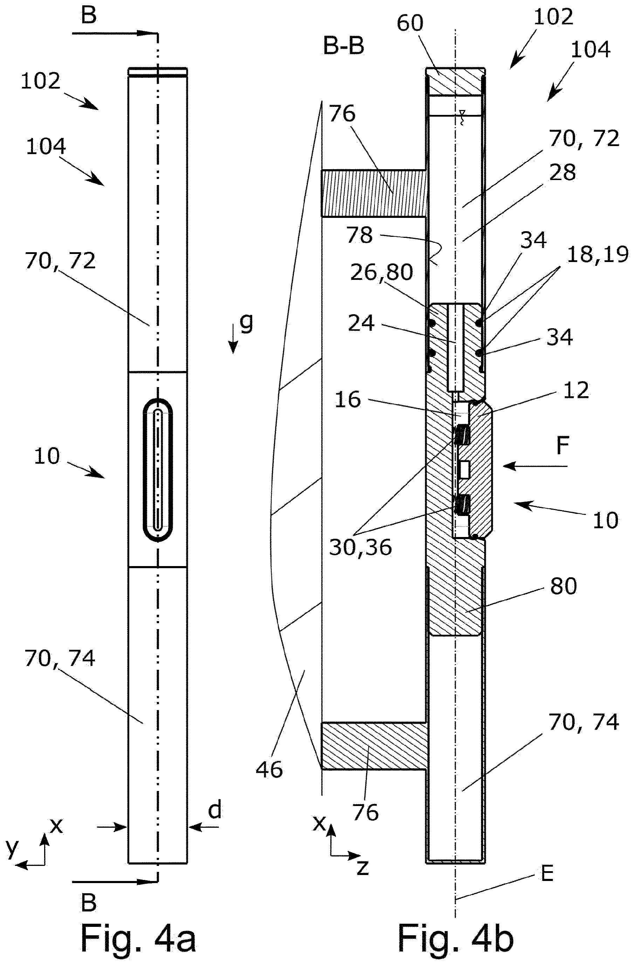

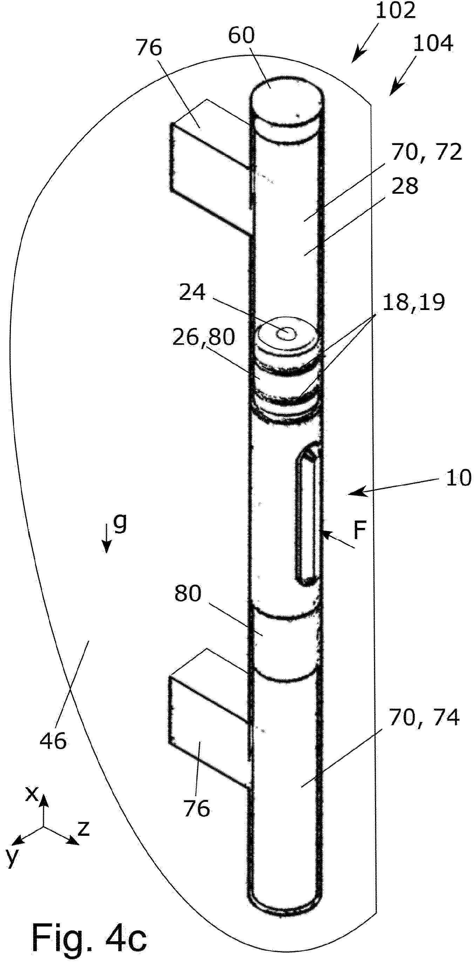

In der Fig. 4a, der Fig. 4b und der Fig. 4c ist der Türgriff 10 als ein Stoßgriff 102 dargestellt, der an einem schematisch dargestellten Türblatt 46 montiert ist. Im Gegensatz zu der vorhergehend beschriebenen Türklinke 100, ist der Stoßgriff 102 fest und der Türgriff 10 nicht beweglich oder drehbar mit dem Türblatt 46 verbunden, wobei sich eine Tür vorzugsweise durch eine Stoßbewegung auf den Stoßgriff 102 öffnen lässt.

Im Vergleich zu dem bereits in der Fig. 1 gezeigten Türgriff 10, bildet der hier gezeigte Türgriff 10 zusammen mit Aufnahmemitteln 70 eine zylindrische oder rohrförmige Griffstange 104 mit einer gemeinsamen Erstreckungsachse E aus, wobei sich das Betätigungselement 12 analog zu der Fig. 1 und der Fig. 2a ebenfalls in der nicht betätigten ersten Stellung befindet.

Der Stoßgriff 102 weist als Aufnahmemittel 70 vorzugsweise einen ersten und einen zweiten rohrförmigen Aufnahmeabschnitt 72, 74 auf, wobei der Türgriff 10 beidseitige männliche oder querschnittsreduzierte Verbindungsabschnitte 80 aufweist und jeweils in den ersten und den zweiten rohrförmigen Aufnahmeabschnitte 72, 74 einsteckbar ist. Vorzugsweise ist dadurch der Türgriff 10 entlang der Griffstange 104 mittig angeordnet.

Vorteilhaferweise weist die Griffstange 104 keine Querschnittsprünge zwischen dem Türgriff 10 und den ersten und zweiten Aufnahmeabschnitten 72, 74 und einen entlang der Erstreckungsachse E kontinuierlichen Außendurchmesser d auf, sodass die Griffstange 104 besonders leicht reinigbar ist und sich keine Verunreinigungen in dem Übergang von dem Türgriff 10 zu dem ersten und zweiten Aufnahmeabschnitten 72, 74 ansammeln können.

Vorzugsweise weist die Griffstange 104 eine Länge von 250mm bis 800mm auf, besonders bevorzugt von 400mm, und einen Durchmesser d von 18mm bis 55mm auf, besonders bevorzugt von 30mm, wobei eine Bedienperson die Griffstange 104 mit einer Hand zumindest teilweise umgreifen können sollte.

Wie insbesondere aus der Fig. 4b ersichtlich wird, ist der erste rohrförmige Aufnahmeabschnitt 72 als Behälter 28 ausgebildet, um eine Flüssigkeit, insbesondere ein Desinfektionsmittel, aufzunehmen. Dabei ist der erste Aufnahmeabschnitt 72 einends mit dem Verbindungsmittel 26 des Türgriffs 10 flüssigkeitsdicht verbunden. Dazu sind Dichtelemente 18, insbesondere zwei O-Ringe 19 in jeweils einer Führungsnut 34, zwischen dem Verbindungsmittel 26 des Türgriffs 10, das gleichzeitig als querschnittsreduzierter und zylindrischer Verbindungsabschnitt 80 ausgebildet ist, und der Innenseite 78 des ersten Aufnahmeabschnitts 72 angeordnet und zur Abdichtung eingepresst. Das Verbindungsmittel 26 ist von dem Kanal 24 durchdrungen, um Flüssigkeit aus dem Behälter 28 in den Hohlraum 16 des Türgriffs 10 zu leiten. Vorzugsweise ist der als Verbindungsmittel 26 ausgebildete querschnittsreduzierte Verbindungsabschnitt 80 in Richtung des Hohlraums 16 aufgebohrt, um den Kanal 24 entlang der Erstreckungsachse E auszubilden.

Im Vergleich zu der Ausführungsform in der Fig. 1 ist der dort dargestellte verjüngte Abschnitt 56 hier als Verbindungsmittel 26 ausgebildet und von dem Kanal 24 durchdrungen.

Insbesondere durch die eingepressten Dichtelemente 18 ist der Türgriff 10 kraftschlüssig mit dem ersten Aufnahmeabschnitt 72 verbunden. Durch den unteren zweiten Aufnahmeabschnitt 74 ist der Türgriff 10 mit dem querschnittsreduzierten Verbindungsabschnitt 80 zusätzlich formschlüssig angeordnet und im montierten Zustand des Stoßgriffes 102 mit dem Türblatt 46 verbunden.

Der erste Aufnahmeabschnitt 72 weist andernends einen Deckel 60 auf, um den als Behälter 28 ausgebildeten ersten Aufnahmeabschnitt 72 zu verschließen. Dadurch kann der Behälter 28 einfach mit der Flüssigkeit befüllt oder nachgefüllt werden.

Der Deckel 60 kann mit dem Behälter 28 kraft- und/oder formschlüssig verbunden sein, wobei ein steckbarer oder schraubbarer Deckel 60 bevorzugt wird. Weiter bevorzugt ist der Deckel 60 durch einen hier nicht dargestellten Sicherungsmechanismus, insbesondere ein verschließbares Schloss, vor einem Diebstahl oder einem ungewollten Befüllen des Behälters gesichert. Alternativ kann als Sicherungsmechanismus der schraubbare Deckel 60 Einkerbungen, insbesondere zwei Bohrungen, bevorzugt Sacklöcher, in einer Stirnseite entlang der Erstreckungsachse E aufweisen, die nurmehr mit einem entsprechenden Spezialwerkzeug bedienbar sind.

Der Behälter weist vorzugsweise ein Volumen von 100ml bis 700ml, besonders bevorzugt von 400ml auf, wobei ein größeres Volumen bevorzugt ist um möglichst viele Betätigungszyklen zu erreichen.

Die Fig. 4a und Fig. 4b zeigen den ersten und den zweiten rohrförmigen Aufnahmeabschnitt 72, 74 mit jeweils einem Montageelement 76, um die Griffstange 104 an das Türblatt 46 zu montieren. Ein Abstand zwischen der Griffstange 104 und dem Türblatt 46 ist derart durch die Montageelemente 76 ausgebildet, dass eine Bedienperson die Griffstange 104 händisch umgreifen kann.

Vorzugsweise ist die Erstreckungsachse E der Griffstange 104 entlang der Richtung der Gewichtskraft g ausgerichtet, wobei der erste Aufnahmeabschnitt 72 sich derart oberhalb des Türgriffs 10 befindet, dass die Flüssigkeit in dem Behälter 28 durch den Kanal 24 in den Hohlraum 16 fließt und vorzugsweise kein komplexer Pumpmechanismus erforderlich ist.

Der Türgriff 10 mit Betätigungselement 12 ist in der Fig. 4b im Wesentlichen analog zu der Fig. 2a dargestellt und analog zu der bereits aufgeführten Figurenbeschreibung bedienbar, wobei das Betätigungselement 12 hier vorzugsweise mit der Kraft F, insbesondere eine Stoßkraft entgegen das Türblatt 46 und Rückstellmittel 30, insbesondere zwei Druckfedern 36, in eine Richtung senkrecht zu der Richtung der Gewichtskraft g betätigbar ist.

Im Unterschied zu der Fig. 1 und der Fig. 2a weist der hier dargestellte Türgriff 10 zur Montage als Stoßgriff 102 beidseitig querschnittsreduzierte Verbindungsabschnitte 80 auf, wobei der Behälter 28 entlang der Erstreckungsachse E oder der Längsmittelachse 50 des Türgriffs mit dem Türgriff 10 verbunden ist und der Kanal 24 den Türgriff 10 und das Verbindungsmittel 26 entlang dieser Erstreckungsachse E durchdringt.

Das Betätigungselement 12 insbesondere derart an der Griffstange 104 den Montageelementen 76 gegenüberliegend angeordnet, insbesondere durch Rotation des Türgriffs 10 um die Erstreckungsachse E, dass das Betätigungselement 12 durch die Druckkraft F in Form einer händisch eingegebenen Stoßkraft, insbesondere mit einem Handballen einer Bedienperson, bedienbar ist und gleichzeitig die Tür durch eine Stoßbewegung geöffnet werden kann.

Besonders bevorzugt entspricht die Druckkraft F auf den Türgriff 10 zur Betätigung des Stoßgriffs 102, insbesondere um eine Tür entgegen ein Rückstellmittel in einem Türscharnier zu bewegen, wenigstens einer Kraft, um das Betätigungselement 12 entgegen die Kraft der Rückstellelemente 30 in die dritte Stellung zu verschieben. Dadurch wird eine Art Auslaufsicherung erreicht, da der Hohlraum 16 und/oder der Kanal 24 in der dritten Stellung des Betätigungselements 12 geschlossen sind.

Alternativ kann der Türgriff 10 auch um die Erstreckungsachse E um 180° gedreht angeordnet sein, wobei dann das Betätigungselement 12 vorzugsweise durch eine Zugkraft, insbesondere mit den Fingern der Hand einer Bedienperson, bedienbar ist.

### Bezugszeichenliste

- 10: Türgriff
- 12: Betätigungselement
- 14: Grifffläche
- 16: Hohlraum
- 18: Dichtelement
- 19: O-Ring
- 20: Erste Dichtfläche des Betätigungselements
- 22: Zweite Dichtfläche des Türgriffs
- 24: Kanal
- 26: Verbindungsmittel
- 28: Behälter
- 30: Rückstellmittel
- 32: Aussparung
- 34: Nut
- 36: Federn
- 38: Vertiefung im Betätigungselement
- 42: Unterseite des Betätigungselements
- 43: Boden des Hohlraums
- 44: Versperrabschnitt
- 46: Türblatt
- 48: Auslassabschnitt eines Behälters
- 50: Längsmittelachse
- 52: Spalt
- 54: Einkerbung im Betätigungselement
- 56: Verjüngter Abschnitt des Türgriffs
- 58: Montageelement der Türklinke
- 60: Deckel des Behälters
- 70: rohrförmiger Aufnahmeabschnitt
- 72: erster Aufnahmeabschnitt
- 74: zweiter Aufnahmeabschnitt
- 76: Montageelement des Stoßgriffs
- 78: Innenseite des ersten Aufnahmeabschnitts
- 80: querschnittsreduzierter Verbindungsabschnitt des Türgriffs

- 100: Türklinke
- 102: Stoßgriff
- 104: Griffstange
- E: Erstreckungsachse der Griffstange 104
- b, l: Breite und Länge des Hohlraums
- d: Durchmesser des Türgriffs
- t: Spaltabstand zwischen den Dichtflächen
- F: Druckkraft einer Bedienperson auf das Betätigungselement
- h: Höhe des Betätigungselements
- g: Richtung der Schwerkraft
- x, y, z: Raumachsen eines kartesischen Koordinatensystems

## Patentansprüche

1. Türgriff, insbesondere für Innen und/oder Außenraumtüren in öffentlichen Einrichtungen und/oder Unternehmen, mit Betätigungselement (12) zur Desinfektion einer Grifffläche (14) des Türgriffs (10), wobei das Betätigungselement (12) zumindest teilweise in dem Türgriff (10) angeordnet ist, das in einer nicht betätigten ersten Stellung einen Hohlraum (16) im Inneren des Türgriffs (10) begrenzt, wobei der Türgriff (10) von einem Kanal (24) durchdrungen ist, der einends in den Hohlraum (16) mündet und andernends Verbindungsmittel (26) zur Verbindung mit einem Behälter (28) aufweist, um über den Kanal (24) und die Verbindungsmittel (26) eine Flüssigkeit, insbesondere ein Desinfektionsmittel, von dem Behälter (28) in den Hohlraum (16) zu leiten, und wobei das Betätigungselement (12) entgegen zumindest ein Rückstellmittel (30) manuell verschiebbar ist,
**dadurch gekennzeichnet,**
**dass** der Hohlraum (16) mit zumindest einem Dichtelement (18) zwischen einer ersten Dichtfläche (20) des Betätigungselements (12) und einer zweiten Dichtfläche (22) des Türgriffs (10) abgedichtet ist, wobei eine Aussparung (32) in zumindest einer der Dichtflächen (20, 22) ausgebildet ist, um in einer das Volumen des Hohlraums (16) gegenüber der ersten Stellung verkleinernden zweiten Stellung des Betätigungselements (12) das Dichtelement (18) von zumindest einer Dichtfläche (20, 22) zu lösen, wobei ein Versperrabschnitt (44) der ersten Dichtfläche (20) so an einem Übergang zwischen dem Hohlraum (16) und dem Kanal (24) angeordnet und ausgerichtet ist, dass der Versperrabschnitt (44) des Betätigungselements (12) den Kanal (24) bei der Überführung des Betätigungselements (12) von der ersten in die zweite Stellung zumindest teilweise schließt oder versperrt, wobei das Betätigungselement (12) die Flüssigkeit aus dem Hohlraum (16) in Richtung der Grifffläche (14) verdrängt und wobei in einer das Volumen des Hohlraums (16) gegenüber der zweiten Stellung weiter verkleinernden dritten Stellung des Betätigungselements (12) das Dichtelement (18) an beiden Dichtflächen (20, 22) anliegt, um den Kanal (24) und/oder den Hohlraum (16) vollständig zu schließen.

2. Türgriff nach Anspruch 1,
**dadurch gekennzeichnet,**
**dass** das Betätigungselement (12) derart zumindest teilweise in dem Türgriff (10) angeordnet ist, dass in einem an einem Türblatt (46) montierten Zustand des Türgriffs (10) eine das Volumen des Hohlraums (16) verkleinernde Stellbewegung des Betätigungselements (12) in Richtung der Schwerkraft (g) ausführbar ist.

3. Türgriff nach einem der Ansprüche 1 oder 2,
**dadurch gekennzeichnet,**
**dass** der Behälter (28) mit einer Auslassseite oder einem Auslassabschnitt (48) auf dem Türgriff (10) flüssigkeitsdicht in Richtung der Schwerkraft (g) verbunden, bevorzugt verschraubt ist.

4. Türgriff nach einem der Ansprüche 1 bis 3,
**dadurch gekennzeichnet,**
**dass** das Dichtelement (18) ein elastischer O-Ring (19) ist und in einer Nut (34) in einer der Dichtflächen (20, 22) eingepasst ist, wobei die Aussparung (32) und die Nut (34) in jeweils einer der Dichtflächen (20, 22) ausgebildet sind.

5. Türgriff nach einem der Ansprüche 1 bis 4,
**dadurch gekennzeichnet,**
**dass** in der ersten Stellung des Betätigungselements (10) eine Haftkraft zwischen Dichtelement (18) und den Dichtflächen (20, 22) größer ist als die Kraft des Rückstellmittels (30), um eine klemmende Befestigung des Betätigungselements (10) im Türgriff (10) zu erreichen.

6. Türgriff nach einem der Ansprüche 1 bis 5,
**dadurch gekennzeichnet,**
**dass** die Rückstellmittel (30) mehrere Federn (36), bevorzugt zwei Federn (36), ganz besonders bevorzugt drei Federn (36), insbesondere Wendelfedern, umfassen, die jeweils in einer Vertiefung (38), insbesondere in einem Sackloch, in dem Betätigungselement (12) geführt sind.

7. Türgriff nach einem der Ansprüche 1 bis 6,
**dadurch gekennzeichnet,**
**dass** der Türgriff (10) als zylindrischer Rundkörper mit einem bevorzugten Durchmesser (d) zwischen 18mm und 22 ausgeführt ist, wobei der Hohlraum (16) bevorzugt als Langloch in dem Türgriff (10) mit einer Länge (l) zwischen 65mm bis 75mm und einer Breite (b) zwischen 10mm bis 14mm ausgebildet ist und wobei ein Spalt (t) zwischen den Dichtflächen (20, 22) und außerhalb der Aussparung (32) zwischen 0.1 mm bis 0.5mm breit ist.

8. Türgriff nach einem der Ansprüche 1 bis 7,
**dadurch gekennzeichnet,**
**dass** das Volumen des Hohlraums (16) in der ersten Stellung des Betätigungselements (12) 2.2cm³ bis 3cm³ entspricht.

9. Türgriff nach einem der Ansprüche 1 bis 8,
**dadurch gekennzeichnet,**
**dass** der Türgriff (10) und das Betätigungselement (12) aus einem Metall, bevorzugt Aluminium oder Edelstahl, gefertigt sind.

10. Türgriff nach einem der Ansprüche 1 bis 9,
**dadurch gekennzeichnet,**
**dass** die Grifffläche (14) eine abrasiv behandelte raue Oberfläche aufweist, bevorzugt mittels Sand- oder Kugelstrahlen, mit einem Rauheitswert zwischen 20µm und 40µm gemessen nach ISO 8503, um den Kontaktwinkel zwischen der Grifffläche (14) und einem Desinfektionsmittel zu vergrößern.

11. Türklinke mit Türdorn (40) und Türgriff (10) mit Betätigungselement (12) nach einem der Ansprüche 1 bis 10,
**dadurch gekennzeichnet,**
**dass** der Türgriff (10) rechtwinklig an den Türdorn (40) montiert ist und gegen ein Verdrehen um eine Längsmittelachse (50) des Türgriffs (10) verschraubt ist.

12. Türklinke nach Anspruch 11,
**dadurch gekennzeichnet,**
**dass** eine Druckkraft (F) auf den Türgriff (10) zur Betätigung der Türklinke (100), insbesondere zur Verformung einer Drehfeder, wenigstens einer Kraft entspricht, um das Betätigungselement (12) entgegen die Kraft der Rückstellelemente (30) in die dritte Stellung zu verschieben.

13. Tür mit Türklinke nach Anspruch 11 oder 12, wobei der Türgriff (10) ein- oder beidseitig einer Tür montiert ist.

14. Tür nach Anspruch 13, wobei ein Behälter (28) an dem Türblatt (46) angebracht ist und mittels eines Schlauches mit dem Kanal (24) des Türgriffs (10) verbunden ist.

15. Stoßgriff, insbesondere ein Bügelgriff, und Türgriff (10) mit Betätigungselement (12) nach einem der Ansprüche 1 oder 3-10,
**dadurch gekennzeichnet,**
**dass** der Stoßgriff (102) Aufnahmemittel (70) aufweist, die zusammen mit dem Türgriff (10) eine bevorzugt zylindrische oder rohrförmige Griffstange (104) mit einer gemeinsamen Erstreckungsachse E ausbilden, wobei die Aufnahmemittel (70) als ein erster rohrförmiger Aufnahmeabschnitt (72) den Behälter (28) ausbilden, der einends mit dem Verbindungsmittel (26) des Türgriffs (10) verbunden ist.

16. Stoßgriff nach Anspruch 15,
**dadurch gekennzeichnet,**
**dass** der Türgriff (10) einends mit dem ersten rohrförmigen Aufnahmeabschnitt (72) und andernends mit einem zweiten rohrförmigen Aufnahmeabschnitt (74) einsteckbar verbunden ist, wobei das Verbindungsmittel (26) des Türgriffs (10) umfangsseitige Dichtmittel (18) aufweisen, um den ersten rohrförmigen Aufnahmeabschnitt (72) als Behälter (28) abzudichten.

17. Tür mit zumindest einem Stoßgriff (102) nach einem der Ansprüche 15 oder 16, wobei die zylindrische Griffstange (104) des Stoßgriffs (102) mit zumindest einem Montageelement (76) an den Aufnahmemitteln (70) ein- oder beidseitig der Tür montiert ist.

18. Tür nach Anspruch 17,
**dadurch gekennzeichnet,**
**dass** die Erstreckungsachse (E) des Stoßgriffs (102) derart ausgerichtet ist, dass eine Flüssigkeit in dem ersten rohrförmigen Aufnahmeabschnitt (72) als Behälter (28) mittels der Schwerkraft (g) in den Türgriff (10) fließt, wobei die Erstreckungsachse (E) des Stoßgriffs (102) vorzugsweise parallel zu der Richtung der Schwerkraft (g) ausgerichtet ist.

## Claims

1. A door handle, in particular for indoor and/or outdoor doors in public facilities and/or companies, having an actuation element (12) for sanitizing a handle surface (14) of the door handle (10), the actuation element (12) being disposed at least partially in the door handle (10) and delimiting a hollow (16) in the interior of the door handle (10) in a first non-actuated position, the door handle (10) being pierced by a channel (24) which opens into the hollow (16) on one end and has a connector (26) on another end for a connection to a container (28) to guide a liquid, in particular a sanitizer, from the container (28) to the hollow (16) via the channel (24) and the connector (26), the actuation element (12) being manually displaceable against at least one return element (30),
**characterized in that**
the hollow (16) is sealed by means of at least one sealant (18) between a first sealing surface (20) of the actuation element (12) and a second sealing surface (22) of the door handle (10), a cutout (32) being formed in at least one of the sealing surfaces (20, 22) in order to release the sealant (18) from at least one sealing surface (20, 22) in a second position of the actuating element (12) decreasing the volume of the hollow (16) with respect to the first position, a blocking section (44) of the first sealing surface (20) being disposed and oriented such at a transition between the hollow (16) and the channel (24) that the blocking section (44) of the actuation element (12) at least partially closes or blocks the channel (24) when displacing the actuation element (12) from the first to the second position, the actuation element (12) displacing the liquid from the hollow (16) towards the handle surface (16) and the sealant (18) abutting against both sealing surfaces (20, 22) in a third position of the actuation element (12) further reducing the volume of the hollow (16) with respect to the second position in order to entirely close the channel (24) and/or the hollow (16).

2. The door handle according to claim 1,
**characterized in that**
the actuation element (12) is disposed in such a manner at least partially in the door handle (10) that a positioning movement of the actuation element (12) reducing the volume of the hollow (16) is executable in the direction of gravity (g) when the door handle (10) is mounted on a door leaf (46).

3. The door handle according to claim 1 or 2,
**characterized in that**
an outlet side or an outlet section (48) of the container (28) is connected to, preferably screwed in place on, the door handle (10) in a liquid-tight manner in the direction of gravity (g).

4. The door handle according to any one of the claims 1 to 3,
**characterized in that**
the sealant (18) is an elastic O-ring (19) and is fit in a groove (34) in one of the sealing surfaces (20, 22), the cutout (32) and the groove (34) being formed in one of the sealing surfaces (20, 22) each.

5. The door handle according to any one of the claims 1 to 4,
**characterized in that**
an adherence between the sealant (18) and the sealing surfaces (20, 22) is larger in the first position of the actuation element (12) than the force of the return element (30) in order to attain a wedged fixation of the actuation element (10) in the door handle (10).

6. The door handle according to any one of the claims 1 to 5,
**characterized in that**
the return elements (30) comprise several springs (36), preferably two springs (36), particularly preferably three springs (36), in particular spiral coiled springs, which are each guided in an indentation (38), in particular a blind bore, in the actuation element (12).

7. The door handle according to any one of the claims 1 to 6,
**characterized in that**
the door handle (10) is designed as a cylindrical round component having a preferred diameter (d) between 18 mm and 22 mm, the hollow (16) preferably being realized as an oblong hole in the door handle (10) having a length (l) between 65 mm and 75 mm and a breadth (b) between 10 mm and 14 mm and a gap (t) between the sealing surfaces (20, 22) and outside of the cutout (32) being between 0.1 mm and 0.5 mm wide.

8. The door handle according to any one of the claims 1 to 7,
**characterized in that**
the volume of the hollow (16) corresponds to 2.2 cm³ to 3 cm³ in the first position of the actuation element (12).

9. The door handle according to any one of the claims 1 to 8,
**characterized in that**
the door handle (10) and the actuation element (12) are made of metal, preferably aluminum or stainless steel.

10. The door handle according to any one of the claims 1 to 9,
**characterized in that**
the handle surface (14) has a rough surface treated abrasively, preferably by means of sand jets or shot-blasting, having a surface roughness between 20 µm and 40 µm measured according to ISO 8503, in order to enlarge the contact angle between the handle surface (14) and a sanitizer.

11. A door fitting having a bolt (40) and the door handle (10) having an actuation element (12) according to any one of the claims 1 to 10,
**characterized in that**
the door handle (10) is mounted at a right angle on the bolt (40) and is screwed in place to prevent a rotation about a longitudinal center axis (50) of the door handle (10).

12. The door fitting according to claim 11,
**characterized in that**
a pressure (F) on the door handle (10) for actuating the door fitting (100), in particular for deforming a torsion spring, corresponds to at least a force in order to displace the actuation element (12) to the third position against the force of the return elements (30).

13. A door having a door fitting according to claim 11 or 12, wherein the door handle (10) is mounted on one or both sides of a door.

14. The door according to claim 13, wherein a container (28) is fixed to the door leaf (46) and is connected to the channel (24) of the door handle (10) by means of a tube.

15. A pull handle, in particular a bow-shaped handle, and a door handle (10) having an actuation element (12) according to any one of the claims 1 or 3 to 10,
**characterized in that**
the pull handle (102) has accommodators (70) which together with the door handle (10) form a preferably cylindrical or tube-shaped handle bar (104) having a shared extension axis E, the accommodators (70) forming the container (28) as a first tube-shaped accommodating section (72), which is connected to the connectors (26) of the door handle (10) on one end.

16. The pull handle according to claim 15,
**characterized in that**
the door handle (10) is connected in an insertable manner to the first tube-shaped accommodating section (72) on one end and to a second tube-shaped accommodating section (74) on another end, the connectors (26) of the door handle (10) having circumferential sealants (18) in order to seal the first tube-shaped accommodating section (72) as a container (28).

17. A door having at least one pull handle (102) according to claim 15 or 16, wherein the cylindrical handle bar (104) of the pull handle (102) is mounted on the accommodators (70) on one or both sides of the door using at least one mounting element (76).

18. The door according to claim 17,
**characterized in that**
the extension axis (E) of the pull handle (102) is oriented in such a manner that a liquid flows to the door handle (10) in the first tube-shaped accommodating section (72) as a container (28) by means of gravity (g), the extension axis (E) of the pull handle (102) preferably being oriented parallel to the direction of gravity (g).

## Revendications

1. Poignée de porte, notamment pour des portes à l'intérieur et/ou à l'extérieur dans les édifices publics et/ou dans les entreprises, ayant un élément d'actionnement (12) pour la désinfection d'une surface (14) de la poignée de porte (10), l'élément d'actionnement (12) étant disposé au moins partiellement dans la poignée de porte (10) et délimitant une cavité (16) à l'intérieur de la poignée de porte (10) dans une première position non actionnée, la poignée de porte (10) étant percée d'un canal (24) qui débouche dans la cavité (16) à une extrémité et a un moyen de connexion (26) à une autre extrémité pour une connexion à un conteneur (28) afin de guider un liquide, notamment un désinfectant, du conteneur (28) à la cavité (16) via le canal (24) et le moyen de connexion (26), l'élément d'actionnement (12) pouvant être déplacé manuellement contre au moins un élément de rappel (30),
**caractérisée en ce que**
la cavité (16) est scellée au moyen d'au moins un moyen d'étanchéité (18) entre une première surface étanchant (20) de l'élément d'actionnement (12) et une deuxième surface étanchant (22) de la poignée de porte (10), un évidement (32) étant formé dans au moins une des surfaces étanchant (20, 22) afin de libérer le moyen d'étanchéité (18) d'au moins une surface étanchant (20, 22) dans une deuxième position de l'élément d'actionnement (12) diminuant le volume de la cavité (16) par rapport à la première position, une partie de verrouillage (44) de la première surface étanchant (20) étant disposée et orientée à une transition entre la cavité (16) et le canal (24) de telle sorte que la partie de verrouillage (44) de l'élément d'actionnement (12) ferme ou bloque au moins partiellement le canal (24) lors du déplacement de l'élément d'actionnement (12) de la première à la deuxième position, l'élément d'actionnement (12) déplaçant le liquide de la cavité (16) vers la surface de poignée (16) et le moyen d'étanchéité (18) butant contre les deux surfaces étanchant (20, 22) dans une troisième position de l'élément d'actionnement (12) réduisant encore le volume de la cavité (16) par rapport à la deuxième position afin de fermer entièrement le canal (24) et/ou la cavité (16).

2. Poignée de porte selon la revendication 1,
**caractérisée en ce que**
l'élément d'actionnement (12) est disposé au moins partiellement dans la poignée de porte (10) de telle manière qu'un mouvement de positionnement de l'élément d'actionnement (12) réduisant le volume de la cavité (16) est exécutable dans la direction de la gravité (g) lorsque la poignée de porte (10) est montée sur un ouvrant (46).

3. Poignée de porte selon la revendication 1 ou la revendication 2,
**caractérisée en ce que**
un côté de sortie ou à une partie de sortie (48) du conteneur (28) est relié, de préférence vissé, à la poignée de porte (10) de manière étanche aux liquides dans le sens de la gravité (g).

4. Poignée de porte selon l'une quelconque des revendications 1 à 3, **caractérisée en ce que**
le moyen d'étanchéité (18) est un joint torique (19) élastique et est inséré dans une rainure (34) dans l'une des surfaces étanchant (20, 22), l'évidement (32) et la rainure (34) étant formés chacun dans l'une des surfaces étanchant (20, 22).

5. Poignée de porte selon l'une quelconque des revendications 1 à 4, **caractérisée en ce**
**qu'**une adhérence entre le moyen d'étanchéité (18) et les surfaces étanchant (20, 22) est plus grande dans la première position de l'élément d'actionnement (12) que la force de l'élément de rappel (30) afin d'atteindre une fixation coincée de l'élément d'actionnement (10) dans la poignée de porte (10).

6. Poignée de porte selon l'une quelconque des revendications 1 à 5, **caractérisée en ce que**
l'élément de rappels (30) comprend plusieurs ressorts (36), de préférence deux ressorts (36), de manière particulièrement préférée trois ressorts (36), notamment des ressorts hélicoïdaux, qui sont chacun guidés dans une indentation (38), notamment un trou borgne, dans l'élément d'actionnement (12).

7. Poignée de porte selon l'une quelconque des revendications 1 à 6,
**caractérisée en ce que**
la poignée de porte (10) est conçue comme un composant rond cylindrique ayant un diamètre (d) préféré entre 18 mm et 22 mm, la cavité (16) étant de préférence réalisée comme trou oblong dans la poignée de porte (10) ayant une longueur (l) entre 65 mm et 75 mm et une largeur (b) entre 10 mm et 14 mm et un espace (t) entre les surfaces étanchant (20, 22) et à l'extérieur de l'évidement (32) étant entre 0,1 mm et 0,5 mm.

8. Poignée de porte selon l'une quelconque des revendications 1 à 7, **caractérisée en ce que**
le volume de la cavité (16) correspond à 2,2 cm³ à 3 cm³ dans la première position de l'élément d'actionnement (12).

9. Poignée de porte selon l'une quelconque des revendications 1 à 8, **caractérisée en ce que**
la poignée de porte (10) et l'élément d'actionnement (12) sont en métal, de préférence en aluminium ou en acier inoxydable.

10. Poignée de porte selon l'une quelconque des revendications 1 à 9, **caractérisée en ce que**
la surface de poignée (14) a une surface rugueuse traitée abrasivement, de préférence par sablage ou grenaillage, ayant une valeur de rugosité entre 20 µm et 40 µm mesurée selon ISO 8503, afin d'élargir l'angle de contact entre la surface de poignée (14) et un désinfectant.

11. Ferrure de porte ayant un verrou (40) et poignée de porte (10) ayant un élément d'actionnement (12) selon l'une quelconque des revendications 1 à 10,
**caractérisée en ce que**
la poignée de porte (10) est montée à angle droit sur le verrou (40) et est vissée en place pour empêcher une rotation autour d'un axe central longitudinal (50) de la poignée de porte (10).

12. Ferrure de porte selon la revendication 11,
**caractérisée en ce**
**qu'**une pression (F) sur la poignée de porte (10) pour actionner la ferrure de porte (100), notamment pour déformer un ressort de torsion, correspond à au moins une force pour déplacer l'élément d'actionnement (12) vers la troisième position contre la force des éléments de rappel (30).

13. Porte ayant une ferrure de porte selon la revendication 11 ou la revendication 12, dans laquelle la poignée de porte (10) est montée sur un ou les deux côtés d'une porte.

14. Porte selon la revendication 13, dans laquelle un conteneur (28) est fixé à l'ouvrant (46) et est relié au canal (24) de la poignée de porte (10) au moyen d'un tube.

15. Poignée de tirage, notamment une poignée d'étrier, et une poignée de porte (10) ayant un élément d'actionnement (12) selon l'une quelconque des revendications 1 ou les revendications 3 à 10,
**caractérisée en ce que**
la poignée de tirage (102) a des moyens de réception (70) qui, avec la poignée de porte (10), forment une tige de poignée (104) de préférence cylindrique ou en forme de tube et ayant un axe d'extension E commun, les moyens de réception (70) formant le conteneur (28) comme première partie de réception (72) en forme de tube, qui est relié aux moyens de connexion (26) de la poignée de porte (10) à une extrémité.

16. Poignée de tirage selon la revendication 15,
**caractérisée en ce que**
la poignée de porte (10) est connectée de manière insérable à la première partie de réception (72) en forme de tube à une extrémité et à une deuxième partie de réception (74) en forme de tube à une autre extrémité, les moyens de connexion (26) de la poignée de porte (10) ayant des moyens d'étanchéité (18) circonférentiels afin de sceller la première partie de réception (72) en forme de tube en tant que conteneur (28).

17. Porte ayant au moins une poignée de tirage (102) selon la revendication 15 ou la revendication 16, dans laquelle la tige (104) cylindrique de la poignée de tirage (102) est montée sur les moyens de réception (70) d'un ou des deux côtés de la porte à l'aide d'au moins un élément de montage (76).

18. Porte selon la revendication 17,
**caractérisée en ce que**
l'axe d'extension (E) de la poignée de tirage (102) est orienté de telle manière qu'un liquide s'écoule vers la poignée de porte (10) dans la première partie de réception (72) en forme de tube comme conteneur (28) au moyen de la gravité (g), l'axe d'extension (E) de la poignée de tirage (102) étant de préférence orienté parallèlement à la direction de la gravité (g).
